# EUROPÄISCHE PATENTSCHRIFT

(11) **EP 3 406 279 B1**
(45) Veröffentlichungstag und Bekanntmachung des Hinweises auf die Patenterteilung: **11.12.2019**
(21) Anmeldenummer: 18183192.6
(22) Anmeldetag: 18.07.2016
(51) Int. Cl.: A61M 5/158, A61M 39/22, A61M 1/36

(54) **BLUTSTOPP-EINRICHTUNG**
BLOOD STOP DEVICE
DISPOSITIF D'ARRÊT DE SANG

(30) Priorität: 16.07.2015 DE 102015009190
(43) Veröffentlichungstag der Anmeldung: 28.11.2018
(62) Teilanmeldung aus: 16739186.1
(73) Patentinhaber: B. Braun Avitum AG, 34212 Melsungen (DE)
(72) Erfinder: HAINDL, Hans, 30974 Wennigsen (DE); WOEHR, Kevin, 34587 Felsberg (DE)
(74) Vertreter: Winter, Brandl, Fürniss, Hübner, Röss, Kaiser, Polte - Partnerschaft mbB

(56) Entgegenhaltungen:
- DE-A1-102012 206 557
- US-A1- 2002 161 333

## Beschreibung

Die vorliegende Anmeldung betrifft eine Blutstopp-Einrichtung für die extrakorporale Blutbehandlung wie zum Beispiel für die Dialyse.

Die Unfallverhütungsvorschrift TRBA 250 fordert, dass an allen medizinischen Arbeitsplätzen, bei denen eine Verletzungsgefahr für Anwender von der Handhabung von Kanülen ausgeht, soweit verfügbar, Sicherheitskanülen eingesetzt werden, die nach Gebrauch aktiv oder passiv so geschützt sind, dass sie den Anwender oder Dritte nicht mehr verletzen können. Diese Forderung gilt unter anderem auch für Dialyse-Stationen und Dialyse-Zentren. Bei der Dialyse wird ein künstlich unter der Haut angelegtes Gefäß, ein sogenannter Shunt, zumeist mit zwei Kanülen punktiert. Diese Kanülen sind großlumig und enthalten daher relativ große Blutmengen, so dass bei der Verletzung des Anwenders mit diesen Kanülen die Infektionsgefahr als besonders hoch einzuschätzen ist. Dazu kommt, dass die Häufigkeit durch Blut übertragbarer Infektionen, wie etwa Hepatitis B und C sowie HIV, unter Dialyse-Patienten deutlich höher ist als im Durchschnitt der Bevölkerung.

Um die Infektionsgefahr hier zu vermindern, wurden bereits sogenannte Sicherheitskanülen für die Dialyse vorgeschlagen. Das Prinzip dieser Sicherheitskanülen beruht darauf, dass nach Gebrauch der Kanüle, jedoch vor dem Entfernen des mit der Haut des Patienten verklebten Kanülenansatzes die Kanüle durch Ziehen am Schlauchansatz der Kanüle aus dem Blutgefäß entfernt wird und in den Kanülenansatz hineingezogen wird. Dort rastet die Kanüle dann ein, so dass die Kanülenspitze nicht wieder aus dem Kanülenansatz hervortreten kann.

Bedauerlicherweise hat es mit diesen Sicherheitskanülen schwerwiegende Zwischenfälle gegeben, die dadurch entstanden sind, dass der Schlauch an der Kanüle nicht - wie vom Hersteller gefordert - separat mit Pflaster auf der Haut des Patienten befestigt worden ist. Dadurch konnten z.B. durch Bewegung des Patienten Zugkräfte auf den Schlauch wirken, was dazu führen konnte, dass die Kanüle während der Dialyse in ihre Schutzvorrichtung zurückgezogen wurde. Dadurch wurde bei der venösen Kanüle das Blut aus der Dialysemaschine nicht in den Patienten zurückgefördert, sondern in die Umgebung. Da der Ausfluss des Blutes nicht behindert wird und es damit zu keiner Drucksteigerung im venösen Schlauchschenkel, auch als venöse Seite bezeichnet, kommt, pumpt die Dialysemaschine in einem solchen Fall weiter, ohne Alarm zu geben. Dies kann zum Verbluten des Patienten führen.

Die aufgetretenen Zwischenfälle mit den Sicherheitskanülen beleuchten ein Problem der Dialyse, das von Beginn an bestanden hat und bis heute nicht gelöst ist. Dies ist die Sicherheitsüberwachung des venösen Schenkels der Dialyse. Die Dialysemaschinen sind mit sensiblen Sensoren versehen, die Störungen im Ablauf der Dialyse wahrnehmen und die Maschine gegebenenfalls abschalten und/oder Alarm geben. Wenn etwa die arterielle Kanüle herausrutscht, so wird zunächst, solange die Kanülenspitze noch im Gewebe liegt, die Kanüle kein Blut mehr ansaugen können, und es kommt zum Unterdruck im arteriellen Schlauchschenkel, auch als arterielle Seite bezeichnet. Dies registriert die Dialysemaschine, sie schaltet sich ab und gibt Alarm. Rutscht die arterielle Kanüle komplett aus dem Gewebe heraus, so zieht sie Luft statt Blut. Dies wird in der Maschine sofort z.B. durch einen Ultraschall-Luftdetektor bemerkt, die Maschine schaltet sich aus und gibt Alarm. Insofern ist der arterielle Schenkel automatisch überwacht.

Anders ist dies beim venösen Schlauchschenkel. Wenn die venöse Kanüle verrutscht und mit der Spitze im Gewebe, also zwischen Shunt und Haut liegt, wird sie durch den gepumpten Blutstrom sofort komplett aus dem Gewebe ausgeworfen, und der Blutstrom entleert sich in die Umgebung. Dabei treten in der Regel nur so kleine und kurzfristige Druckänderungen auf, dass der Drucksensor des Dialyse-Gerätes diese häufig nicht wahrnehmen kann. Deshalb hat es durch das Herausrutschen der venösen Kanüle auch schon zahlreiche Todesfälle durch Verbluten der Dialyse-Patienten gegeben. Bei den verwendeten Förderraten kann der Patient nach drei bis fünf Minuten tot sein.

Im Stand der Technik sind verschiedene Verfahren und Vorrichtungen beschrieben, mittels derer der korrekte Sitz einer Kanüle überwacht werden kann, vgl. z.B. DE 10 2009 004 018 A1, US 2006/0130591 A1, DE 199 53 068 A1 und WO 99/24145. Diese Verfahren und Vorrichtungen sind jedoch ausgesprochen komplex und verlangen insbesondere nach einer speziellen Sensorik. Dementsprechend sind diese bekannten Verfahren und Vorrichtungen teuer und wenig praktikabel.

Ferner ist aus der DE 10 2012 206 557 A1 eine Sicherheitskanüle bekannt, welche eine Sicherheitseinrichtung aufweist, die dazu geeignet ist, bei Ablösen des Kanülengehäuses von der Haut des Patienten automatisch die Punktionskanüle in eine geschützte Position in das Gehäuse zurückzuziehen. Eine distale Öffnung des Kanülengehäuses kann dabei mit Hilfe eines Abdeckelements teilweise verschlossen werden, um eine Hemmung des Blutflusses zu bewirken, der einen von einem Dialysegerät registrierbaren Druckanstieg verursacht. Allerdings wird bei dieser Sicherheitskanüle die Kanüle durch die Sicherheitseinrichtung aktiv bewegt, was insofern nachteilig sein kann, als sie dann unter Umständen aus dem Blutgefäß gezogen wird, selbst wenn sie noch ordnungsgemäß darin lag. Darüber hinaus ist der Verschlussmechanismus mit Hilfe des zusätzlichen Abdeckelements relativ kompliziert, da er einerseits eines Mechanismus zum Schutz der Kanüle und andererseits eines Mechanismus zum Generieren des Blutstaus bedarf.

Aus der US 2002/161333 A1 ist eine Blutstopp-Einrichtung gemäß der Präambel des Anspruchs 1 bekannt. In dieser Blutstopp-Einrichtung wird eine schlauchartige Kanüle durch einen Klemm-/ Quetschkörper abgeklemmt, wenn an der Blutstopp-Einrichtung keine medizinische Vorrichtung angebracht ist. Die schlauchartige Kanüle wird von dem Klemm-/ Quetschkörper nicht abgeklemmt, wenn an der Blutstopp-Einrichtung eine medizinische Vorrichtung angebracht ist.

Es ist daher eine Aufgabe der vorliegenden Erfindung, den oben genannten Sicherheitsanforderungen auf einfache Weise, aber dennoch zuverlässig Rechnung zu tragen.

Diese Aufgabe wird mit einer Blutstopp-Einrichtung gemäß Anspruch 1 gelöst. Weitere bevorzugte Merkmale sind in den abhängigen Ansprüchen beschrieben.

Die vorliegende Erfindung betrifft die Bereitstellung eines Blutstopps bzw. Blutstopp-Einrichtung, der vorzugsweise mit einer Sicherheitskanüle eine (mechanisch starre) Einheit bilden kann, aufweisend einen Klemm-/Quetschkörper, der gegen einen flexiblen Schlauch (mittels einer Feder) vorgespannt ist und einem mit dem Klemm-/Quetschkörper in Wirkeingriff stehenden oder bringbaren Auslösehebel oder Auslöseknopf (der am/im Gehäuse des Blutstopps gelagert ist), der so angeordnet ist, dass er bei bzw. durch Aufbringen des Blutstopps (dessen Gehäuses) auf einer Oberfläche, vorzugsweise einer Patientenhaut in eine erste Position bewegt wird (durch Aufdrücken des Gehäuses auf die Oberfläche), in welcher er den Klemm-/Quetschkörper vom Schlauch beabstandet und der sich bei Abheben des Klemm-/Quetschkörpers von der Oberfläche selbsttätig (aufgrund einer Federvorspannung) in eine zweite Position bewegt, in welcher der Klemm-/Quetschkörper mit dem Schlauch in Quetscheingriff kommt (und diesen sperrt).

Bevorzugte Ausführungsformen der erfindungsgemäßen Blutstopp-Einrichtung werden nachfolgend mit Bezug auf die Figuren im Detail beschrieben. Es zeigen:
Fig. 1 eine seitliche Schnittansicht einer Schlauchklemme gemäß einer ersten bevorzugten Ausführungsform der vorliegenden Erfindung; und
Fig. 2 eine seitliche Schnittansicht einer Schlauchklemme gemäß einer zweiten bevorzugten Ausführungsform der vorliegenden Erfindung.

Grundsätzlich hat ein Blutstopp gemäß der vorliegenden Erfindung ein Gehäuse 100, durch welches ein flexibler Blutschlauch 102 hindurchgeführt ist. In dem Gehäuse 100 ist ferner ein Klemm-/Quetschkörper 104 gelagert, der gegen den Blutschlauch 102 federvorgespannt ist. Der Klemm-/Quetschkörper 104 hat eine Eingriffskante/-seite 108, die dafür angepasst ist, den Blutschlauch 102 nahezu fluiddicht abzuquetschen, wofür die auf den Klemm-/Quetschkörper 104 einwirkende Vorspannkraft einer Vorspannfeder 106 ausreicht.

An dem Gehäuse 100 ist ein Auslöseknopf oder Auslösehebel 110 gelagert, der aus dem Gehäuse 100 herausragt bzw. an einer Gehäuseaußenseite gelagert ist und gegen die Gehäuseaußenseite bzw. in das Gehäuseinnere durch eine äußere Kraftbeaufschlagung bewegt werden kann. Der Auslöseknopf oder Auslösehebel 110 ist dabei mit Klemm-/Quetschkörper 104 in Wirkeingriff, derart, dass er diesen in einer ersten, nicht klemmenden/nicht quetschenden Position zurückhält, wenn er in/an das Gehäuse 100 bewegt ist und den Klemm-/Quetschkörper 104 für dessen Bewegen in eine zweite, klemmende/quetschende Position freigibt, wenn er sich aus/weg von dem Gehäuse 100 bewegt.

Dies bedeutet, dass wenn das Gehäuse 100 des Blutstopps beispielsweise an einer Patientenhaut aufliegt - und zwar auf jener Gehäuseseite, an der sich der Auslöseknopf/-hebel 110 befindet - so wird der Auslöseknopf/-hebel 110 gegen/in das Gehäuse 100 gedrückt, wobei der Klemm-/Quetschkörper 104 in der ersten Position gehalten wird, in der Blut frei durch den flexiblen Schlauch 102 innerhalb des Gehäuses 100 strömen kann. Wird hingegen das Gehäuse 100 von der Patientenhaut abgehoben, springt der Auslöseknopf/-hebel 110 vorzugsweise federvorgespannt aus/weg von dem Gehäuse 100 und gibt dabei den Klemm-/Quetschkörper 104 derart frei, dass dieser durch dessen Federvorspannung gegen den im Gehäuse 100 befindlichen flexiblen Schlauch 102 gedrückt wird und diesen sperrt.

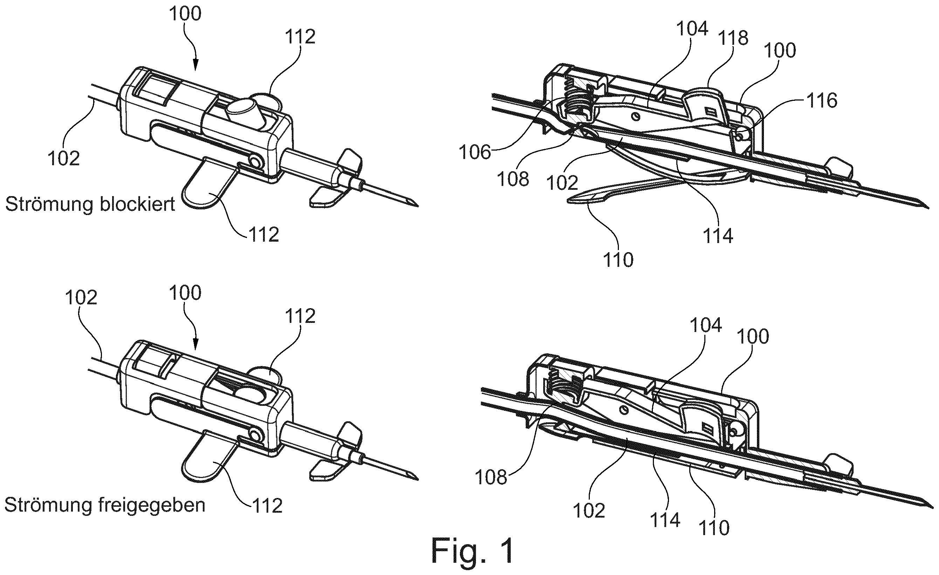

In der Fig. 1 ist eine erste konstruktive Ausführungsform eines Blutstopps gemäß der vorliegenden Erfindung dargestellt.

Demnach hat der erfindungsgemäße Blutstopp das Gehäuse 100, an dem beidseits Patientenhaut-Auflagepads 112 ausgebildet/angeformt sind, deren Patientenhaut-zugewandte Auflageflächen mit Selbstklebestreifen (nicht dargestellt) versehen sind. Das Gehäuse 100 bildet dabei einen Durchgangskanal 114, in welchen der flexible (Blut-)Schlauch 102 eingelegt bzw. einlegbar ist.

An einer Patientenhaut-zugewandten Gehäuseseite ist der Auslösehebel 110 am Gehäuse 100 angelenkt, an dem ein Verraststift 116 ausgeformt/angeordnet ist, der in das Gehäuse 100 hineinragt und mit dem, vorliegend wippenartig im Gehäuse gelagerten Klemmkörper 104 in Rasteingriff bringbar ist.

Im Konkreten ist ein (dem Verraststift 116 abgewandtes) freies Ende des wippenartigen Klemmkörpers 104 mit der Quetschkante 108 ausgebildet, welche mittels der Feder (Schraubenfeder) 106 gegen den Durchgangskanal 114 bzw. den darin eingelegten Schlauch 102 vorgespannt ist. Das andere freie Ende des wippenartigen Klemmkörpers 104 ist mit einer Rasteinrichtung (z.B. in Form eines Rastvorsprungs, jedoch nicht im Detail dargestellt) ausgeformt, die mit dem Verraststift 116 (z.B. in Form einer Rastnase oder Hakens) in Rasteingriff bringbar ist, um den wippenartigen Klemmkörper 104 in der ersten Position zu halten, in der sich die Quetschkante 108 entfernt vom Durchgangskanal 114 (vom flexiblen Schlauch 102) befindet. Der Rasteingriff ist nur dann möglich, wenn der Auslösehebel 110 gegen die entsprechende Gehäuseseite geschwenkt ist (und damit der Verraststift 116 gegen die Rasteinrichtung gedrückt wird).

Schließlich ist an dem anderen freien Ende des wippenartigen Klemmkörpers 104 (im Bereich der Rasteinrichtung) ein weiterer Betätigungsknopf oder Taste 118 angeordnet/ausgebildet, der aus dem Gehäuse 100 ragt, derart, dass bei dessen manueller Betätigung der wippenartige Klemmkörper 104 aus der zweiten Position, in welcher die Quetschkante 108 gegen den eingelegten Schlauch 102 mittels der Feder 106 gedrückt wird, in die erste Position gegen die Federvorspannung zurückbewegt werden kann, in welcher er mit dem Auslösehebel 110 bzw. dem daran ausgebildeten Raststift 116 in Rasteingriff bringbar ist.

Die Funktion des erfindungsgemäßen Blutstopps lässt sich wie Folgt umschreiben. Zunächst wird das Gehäuse 100 auf eine Patientenhaut aufgeklebt, wodurch der Auslösehebel 110 gegen die Gehäuseaußenseite (federelastisch - siehe die am Ausklösehebel 110 angeordnete Blattfeder gemäß Fig. 1) verschwenkt wird. Anschließend wird der wippenartige Klemmkörper 104 über den Betätigungsknopf 118 in dessen erste Position überführt, in welcher er mit Verraststift 116 am Auslösehebel 110 verrastet. Schließlich wird der flexible Schlauch 102 in den Durchgangskanal 114 eingeschoben.

Sollte nunmehr das Gehäuse 100 des Blutstopps von der Patientenhaut abgelöst/abgehoben werden, schwenkt der Auslösehebel 110 federelastisch von der Gehäuseseite weg, wobei auch der Verraststift 116 mitschwenkt und dabei die Verrastung mit dem wippenartigen Klemmkörper 104 löst. Dieser wird nunmehr infolge der darauf einwirkenden Federkraft durch die Feder 106 ebenfalls wippenartig verschwenkt, wobei dessen Quetschkante 108 gegen den Schlauch 102 gedrückt wird und diesen sperrt.

Sofern der Blutstopp mit der erfindungsgemäßen Sicherheitskanüle vorzugsweise gemäß der vorstehenden Beschreibung gehäuseseitig gekoppelt ist (zu einer, vorzugsweise starren Einheit zusammengebaut oder ausgeformt), würde ein Ablösen der Sicherheitskanüle von der Patientenhaut gleichsam ein Ablösen des Gehäuses 100 des Blutstopps von der Patientenhaut bedeuten, wodurch der vorstehend beschriebene Mechanismus (unabhängig von der Sicherheitskanüle) ausgelöst werden würde.

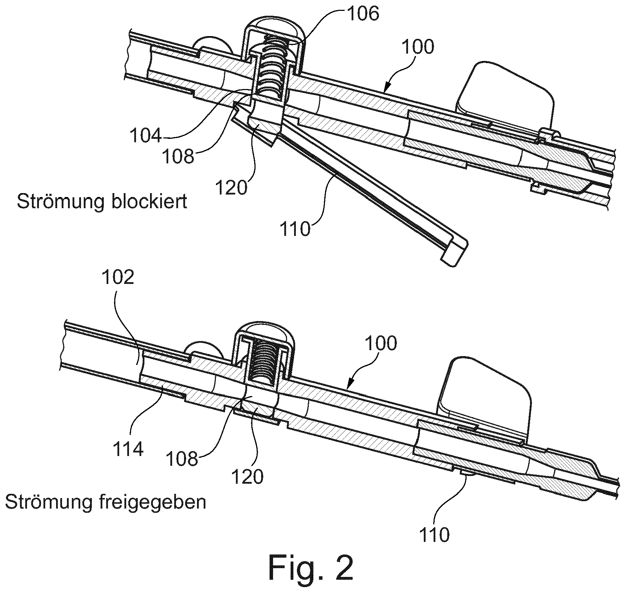

Eine konstruktiv andere Variante des erfindungsgemäßen Blutstopps (Sperrventils) ist in der Fig. 2 dargestellt.

Auch in diesem Fall ist ein Auslösehebel 110 an einer Patientenhaut-zugewandten Seite des Gehäuses 100 des Blutstopps angelenkt. In diesem Fall ist der Klemmkörper 104 aber in Form eines federvorgespannten Stifts ausgebildet, an dessen einer Stirnseite die Vorspannfeder 106 eine axial gerichtete Vorspannkraft aufbringt und an dessen anderer Stirnseite die Quetschkante 108 ausgeformt ist.

In einem (der Feder 106 axial abgewandten) Abschnitt des Stifts 104 ist ein Eingriffselement 120 in der Form einer Halteleiste ausgebildet, an welcher der Auslösehebel 110 nach Art eines Kniehebelmechanismus angreift, um den Stift 104 in die erste Position (weg vom Durchgangskanal 114/eingelegten flexiblen Schlauch 102) zu verschieben, wenn der Auslösehebel 110 gegen die Gehäuseseite verschwenkt wird. Insoweit ist die Funktion des Blutstopps in der Ausführungsform gemäß der Fig. 2 dieselbe, wie die des Ausführungsbeispiels gemäß der Fig. 1 mit der Ausnahme, dass die erste Position des nunmehr stiftförmigen Klemmkörpers 104 beim Ausführungsbeispiel gemäß der Fig. 2 nicht verrastet wird sondern über die Schwenkposition des Auslösehebels 110 sowie die darauf kontinuierlich aufgebrachte Kraft gehalten werden muss.

Die Ausführungsform gemäß der Fig. 2 ist daher nicht notwendiger Weise als konstruktiv einfachere Variante der Ausführungsform gemäß Fig 1 gedacht. Vielmehr wird hier der Effekt angestrebt, dass der Mechanismus nicht erst "scharfschalten" oder "aktivieren" muss, wie dies bei der Ausführungsform gemäß der Fig. 1 der Fall ist.

In anderen Worten ausgedrückt, wenn der Mechanismus / das Gehäuse des Blutstopps auf der Haut eine Patienten klebt, dann wird der Auslösehebel 110 zwangsweise an das Gehäuse 100 des Blutstopps geführt und somit der Teil / das Eingriffselement 120 in das Gehäuse 100 verschoben, um den Blutweg freizugeben. Wird nun das Gehäuse 100 von der Patientenhaut abgelöst, wird durch die Feder 106 das Teil / das Eingriffselement 120 wieder (aus dem Gehäuse 100 heraus) verschoben, sodass kein Blutfluss stattfinden kann. Eine alternative, erfindungsgemäße Idee kann hier sein, dass nicht das Abklemmen eines Schlauches erreicht werden soll, sondern die "Verschiebung" eines Gehäusesegmentes bewirkt wird, das Teil des Blutwegs darstellt und somit wie der Ventilkolben eines Sperrventils der Schiebeventilbauart wirkt. Natürlich muss hierbei die Abdichtung zwischen dem Eingriffselement 120 und dem Gehäuse 100 passen, damit das System funktioniert und es keine Undichtigkeiten gibt.

## Patentansprüche

1. Blutstopp-Einrichtung aufweisend einen Klemm-/ Quetschkörper (104), welcher gegen einen flexiblen Schlauch (102) vorgespannt ist, **gekennzeichnet durch** einen mit dem Klemm-/ Quetschkörper (104) in Wirkeingriff stehenden oder bringbaren Auslösehebel (110) oder Auslöseknopf, welcher so angeordnet ist, dass er bei oder durch Aufbringen der Blutstopp-Einrichtung auf einer Oberfläche in eine erste Position bewegt wird, in welcher er den Klemm-/ Quetschkörper (104) von dem Schlauch (102) beabstandet, und der sich bei Abheben des Klemm-/ Quetschkörpers (104) von der Oberfläche selbsttätig in eine zweite Position bewegt, in welcher der Klemm-/ Quetschkörper (104) mit dem Schlauch (102) in Quetscheingriff kommt.

2. Blutstopp-Einrichtung nach Anspruch 1, **gekennzeichnet durch** ein Gehäuse (100), durch welches der flexible Schlauch (102) hindurchgeführt ist, und in welchem der Klemm-/ Quetschkörper (104) gelagert ist.

3. Blutstopp-Einrichtung nach Anspruch 1 oder 2, **dadurch gekennzeichnet, dass** der Klemm-/ Quetschkörper (104) eine Eingriffskante (108) hat, die dafür angepasst ist, den flexiblen Schlauch (102) durch eine auf den Klemm-/ Quetschkörper (104) einwirkende Vorspannkraft einer Feder (106) nahezu fluiddicht abzuquetschen.

4. Blutstopp-Einrichtung nach einem der vorhergehenden Ansprüche, **dadurch gekennzeichnet, dass** der Auslösehebel (110) oder Auslöseknopf an oder in dem Gehäuse (100) gelagert ist, wobei der Auslösehebel (110) oder Auslöseknopf aus dem Gehäuse (100) herausragt und gegen die Gehäuseaußenseite oder in das Gehäuseinnere durch eine äußere Kraftbeaufschlagung bewegt werden kann.

5. Blutstopp-Einrichtung nach einem der vorhergehenden Ansprüche, **dadurch gekennzeichnet, dass** der Auslösehebel (110) oder Auslöseknopf mit dem Klemm-/ Quetschkörper (104) derart in Wirkeingriff ist, dass er diesen in einer ersten, nicht klemmenden oder nicht quetschenden Position zurückhält, wenn er in oder an das Gehäuse (100) bewegt ist, und den Klemm-/ Quetschkörper (104) für dessen Bewegen in eine zweite, klemmende oder quetschende Position freigibt, wenn er sich aus oder weg von dem Gehäuse (100) bewegt.

6. Blutstopp-Einrichtung nach einem der vorhergehenden Ansprüche, **dadurch gekennzeichnet, dass** der Klemm-/ Quetschkörper (104) in der ersten Position mit dem Auslösehebel (110) oder Auslöseknopf in Rasteingriff bringbar ist.

7. Blutstopp-Einrichtung nach einem der vorhergehenden Ansprüche, **dadurch gekennzeichnet, dass** an dem Auslösehebel (110) oder Auslöseknopf ein in das Gehäuse (100) hineinragender Verraststift (116) ausgeformt ist, mit welchem der wippenartig im Gehäuse (100) gelagerte Klemm-/ Quetschkörper (104), welcher an einem ersten freien Ende mit einer Rasteinrichtung ausgeformt ist, in Rasteingriff bringbar ist.

8. Blutstopp-Einrichtung nach Anspruch 6 oder 7, **dadurch gekennzeichnet, dass** der Rasteingriff nur möglich ist, wenn der Auslösehebel (110) oder Auslöseknopf gegen die der Patientenhaut-zugewandten Gehäuseseite geschwenkt ist und der Verraststift (116) gegen die Rasteinrichtung gedrückt wird.

9. Blutstopp-Einrichtung nach einem der Ansprüche 6 bis 8, **dadurch gekennzeichnet, dass** an dem ersten freien Ende des wippenartigen Klemm-/ Quetschkörpers (104) im Bereich der Rasteinrichtung ein Betätigungsknopf oder Taste (118) ausgebildet ist, welche/r derart aus dem Gehäuse (100) ragt, dass bei dessen/ deren manueller Betätigung der wippenartige Klemm-/ Quetschkörper (104) aus der zweiten Position in die ersten Position gegen die Federvorspannung zurückbewegt werden kann.

10. Blutstopp-Einrichtung nach einem der vorhergehenden Ansprüche, **dadurch gekennzeichnet, dass** ein zweites freies Ende des wippenartigen Klemm-/ Quetschkörpers (104) mit einer Quetschkante (108) ausgebildet ist, welche mittels einer Feder (106) gegen einen in dem Gehäuse ausgebildeten Durchgangskanal (114) oder den darin eingelegten Schlauch (102) vorgespannt ist.

11. Blutstopp-Einrichtung nach Anspruch 10, **dadurch gekennzeichnet, dass** sich die Quetschkante (108) in der ersten Position entfernt von dem Durchgangskanal (114) oder den darin eingelegten Schlauch (102) befindet und dass in der zweiten Position die Quetschkante (108) gegen den eingelegten Schlauch (102) mittels der Feder (106) gedrückt wird.

12. Blutstopp-Einrichtung nach einem der Ansprüche 1 bis 5, **dadurch gekennzeichnet, dass** ein Blutstopp durch Verschieben eines Gehäusesegments, das einen Teil des Blutwegs darstellt, bewirkt wird.

13. Blutstopp-Einrichtung nach einem der Ansprüche 1 bis 5 oder 12, **dadurch gekennzeichnet, dass** der Klemm-/ Quetschkörper (104) in Form eines federvorgespannten Stifts ausgebildet ist, an dessen einer Stirnseite eine Feder (106) eine axial gerichtete Vorspannkraft aufbringt und an dessen anderer Stirnseite eine Quetschkante (108) ausgeformt ist.

14. Blutstopp-Einrichtung nach Anspruch 13, **dadurch gekennzeichnet, dass** in einem der Feder (106) axial abgewandten Abschnitt des Stifts ein Eingriffselement (120) in der Form einer Halteleiste ausgebildet ist, an welcher der Auslösehebel (110) oder Auslöseknopf nach Art eines Kniehebelmechanismus angreift, um den Stift in die erste Position zu verschieben, wenn der Auslösehebel (110) oder Auslöseknopf gegen die Gehäuseseite verschwenkt wird.

15. Blutstopp-Einrichtung nach einem der vorhergehenden Ansprüche, **dadurch gekennzeichnet, dass** an dem Gehäuse (100) beidseits Patientenhaut-Auflagepads (112) ausgebildet sind, deren Patientenhaut-zugewandte Auflageflächen mit Selbstklebestreifen versehen sind.

## Claims

1. Blood stopping device having a clamping/pinching body (104) which is biased against a flexible hose (102), **characterised by** a release lever (110) or release button which is in operative engagement or can be brought into operative engagement with the clamping/pinching body (104), which release lever or release button is arranged such that during/by applying the blood stopping means on a surface it is moved into a first position in which it keeps the clamping/pinching body (104) spaced from the hose (102) and during lifting the clamping/pinching body (104) from the surface automatically moves to a second position in which the clamping/pinching body (104) pinches off the hose (102).

2. Blood stopping device according to claim 1, **characterised by** a housing (100) through which the flexible hose (102) is guided, and in which the clamping/pinching body (104) is mounted.

3. Blood stopping device according to claim 1 or 2, **characterised in that** the clamping/pinching body (104) has an engagement edge (108) which is adapted to pinch off in a largely fluid-tight manner the flexible hose (102) by means of a biasing force, acting on the clamping/pinching body (104), of a spring (106).

4. Blood stopping device according to any of the preceding claims, **characterised in that** the release lever (110) or release button is mounted on or in the housing (100), wherein the trigger lever (110) or trigger button protrudes out of the housing (100) and can be moved against the housing exterior or into the housing interior by means of an external application of force.

5. Blood stopping device according to any of the preceding claims, **characterised in that** the release lever (110) or release button is in operative engagement with the clamping/pinching body (100) such that it retains said clamping/pinching body in a first, not clamping or not pinching position, when it is moved into or onto the housing (100), and releases the clamping/pinching body (104) for its moving into a second, clamping or pinching position when it moves out of or away from the housing (100).

6. Blood stopping device according to any of the preceding claims, **characterised in that** the clamping/pinching body (104) in the first position can be brought into latching engagement with the release lever (110) or release button.

7. Blood stopping device according to any of the preceding claims, **characterised in that** on the release lever (110) or release button is formed a latching pin (116) protruding into the housing (100), with which latching pin the clamping/pinching body (104), mounted in a rocking manner in the housing (100) and formed on a first free end with a detent mechanism, can be brought into latching engagement.

8. Blood stopping device according to claim 6 or 7, **characterised in that** the latching engagement is only possible when the release lever (110) or release button is swivelled against the housing side facing the patient's skin and the latching pin (116) is pressed against the detent mechanism.

9. Blood stopping device according to any of claims 6 to 8, **characterised in that** on the first free end of the rocker-like clamping/pinching body (104) in the region of the detent mechanism is formed an actuating button or key (118) which projects out of the housing (100) in such a manner that when it is manually actuated the rocker-like clamping/pinching body (104) can be moved out of the second position into the first position against the spring bias.

10. Blood stopping device according to any of the preceding claims, **characterised in that** a second free end of the rocker-like clamping/pinching body (104) is formed with a pinching edge (108) which is biased by means of a spring (106) against a through channel (114) formed in the housing or the hose (102) laid therein.

11. Blood stopping device according to claim 10, **characterised in that** the pinching edge (108) in the first position is situated distanced from the through channel (114) or the hose (102) laid therein and that in the second position the pinching edge (108) is pressed against the inlaid hose (102) by means of the spring (106).

12. Blood stopping device according to any of claims 1 to 5, **characterised in that** a blood stop is effected by moving a housing segment which constitutes a part of the blood path.

13. Blood stopping device according to any of claims 1 to 5 or 12, **characterised in that** the clamping/pinching body (104) is formed in the shape of a spring biased pin, at the one end face of which a spring (106) applies an axially-directed biasing force and at the other end face of which is formed a pinching edge (108).

14. Blood stopping device according to claim 13, **characterised in that** in a portion, facing away axially from the spring (106), of the pin is formed an engagement element (120) in the form of a holding strip , on which the release lever (110) or release button engages in the manner of a toggle mechanism in order to move the pin into the first position, when the release lever (110) or release button is swivelled against the housing side.

15. Blood stopping device according to any of the preceding claims, **characterised in that** patient's skin contact pads (112) are formed on both sides on the housing, the contact surfaces, facing the patient's skin, of which are furnished with self-adhesive strips.

## Revendications

1. Dispositif d'arrêt de sang avec un corps de serrage/d'écrasement (104), lequel est précontraint contre un tuyau flexible (102), **caractérisé par** un levier de déclenchement (110) ou un bouton de déclenchement, coopérant ou pouvant coopérer avec le corps de serrage/d'écrasement (104), lequel est disposé de telle sorte qu'il est déplacé lors ou du fait de l'installation du dispositif d'arrêt de sang sur une surface dans une première position, dans laquelle il espace le corps de serrage/d'écrasement (104) du tuyau flexible (102), et qui se déplace, lors du soulèvement du corps de serrage/d'écrasement (104) de la surface, de manière autonome dans une deuxième position, dans laquelle le corps de serrage/d'écrasement (104) vient en prise par écrasement avec le tuyau flexible (102).

2. Dispositif d'arrêt de sang selon la revendication 1, **caractérisé par** un boîtier (100), à travers lequel le tuyau flexible (102) est guidé et dans lequel le corps de serrage/d'écrasement (104) est monté.

3. Dispositif d'arrêt de sang selon la revendication 1 ou 2, **caractérisé en ce que** le corps de serrage/d'écrasement (104) a une arête de prise (108), qui est adaptée pour fermer de manière quasiment étanche aux fluides par écrasement le tuyau flexible (102) par une force de précontrainte d'un ressort (106), agissant sur le corps de serrage/d'écrasement (104).

4. Dispositif d'arrêt de sang selon l'une quelconque des revendications précédentes, **caractérisé en ce que** le levier de déclenchement (110) ou le bouton de déclenchement est monté au niveau de ou dans le boîtier (100), dans lequel le levier de déclenchement (110) ou le bouton de déclenchement dépasse hors du boîtier (100) et peut être déplacé par l'action exercée par une force extérieure contre le côté extérieur de boîtier ou dans l'intérieur de boîtier.

5. Dispositif d'arrêt de sang selon l'une quelconque des revendications précédentes, **caractérisé en ce que** le levier de déclenchement (110) ou le bouton de déclenchement coopère avec le corps de serrage/d'écrasement (104) de telle manière qu'il retient celui-ci dans une première position n'exerçant ni serrage ni écrasement quand il se déplace dans ou au niveau du boîtier (100) et libère le corps de serrage/d'écrasement (104) en vue de son déplacement dans une deuxième position exerçant une action de serrage ou d'écrasement quand il est déplacé hors du boîtier (100) ou de manière à s'en éloigner.

6. Dispositif d'arrêt de sang selon l'une quelconque des revendications précédentes, **caractérisé en ce que** le corps de serrage/d'écrasement (104) peut être amené en prise par enclenchement dans la première position avec le levier de déclenchement (110) ou le bouton de déclenchement.

7. Dispositif d'arrêt de sang selon l'une quelconque des revendications précédentes, **caractérisé en ce qu'**est formée, au niveau du levier de déclenchement (110) ou du bouton de déclenchement, une tige d'enclenchement (116) dépassant dans le boîtier (100), avec laquelle le corps de serrage/d'écrasement (104) monté à la manière d'une bascule dans le boîtier (100), lequel est formé au niveau d'une première extrémité libre avec un dispositif d'enclenchement, peut être amené en prise par enclenchement.

8. Dispositif d'arrêt de sang selon la revendication 6 ou 7, **caractérisé en ce que** la prise par enclenchement est possible seulement quand le levier d'enclenchement (110) ou le bouton d'enclenchement est pivoté contre le côté de boîtier tourné vers la peau du patient et la tige d'enclenchement (116) est poussée contre le dispositif d'enclenchement.

9. Dispositif d'arrêt de sang selon l'une quelconque des revendications 6 à 8, **caractérisé en ce qu'**est réalisé, au niveau de la première extrémité libre du corps de serrage/d'écrasement (104) de type bascule, dans la zone du dispositif d'enclenchement, un bouton d'activation ou une touche (118), lequel ou laquelle dépasse de telle manière hors du boîtier (100) que lors de son activation manuelle, le corps de serrage/d'écrasement (104) de type bascule peut être ramené de la deuxième position dans la première position à l'encontre de la précontrainte de ressort.

10. Dispositif d'arrêt de sang selon l'une quelconque des revendications précédentes, **caractérisé en ce qu'**une deuxième extrémité libre du corps de serrage/d'écrasement (104) de type bascule est réalisée avec une arête d'écrasement (108), laquelle est précontrainte au moyen d'un ressort (106) contre un canal de passage (114), réalisé dans le boîtier, ou le tuyau flexible (102) inséré dans celui-ci.

11. Dispositif d'arrêt de sang selon la revendication 10, **caractérisé en ce que** l'arête d'écrasement (108) se trouve dans la première position de manière éloignée du canal de passage (114) ou du tuyau flexible (102) inséré dans celui-ci, et que dans la deuxième position, l'arête d'écrasement (108) est poussée au moyen du ressort (106) contre le tuyau flexible (102) inséré.

12. Dispositif d'arrêt de sang selon l'une quelconque des revendications 1 à 5, **caractérisé en ce qu'**un arrêt de sang est provoqué par le coulissement d'un segment de boîtier, qui constitue une partie du circuit sanguin.

13. Dispositif d'arrêt de sang selon l'une quelconque des revendications 1 à 5 ou 12, **caractérisé en ce que** le corps de serrage/d'écrasement (104) est réalisé sous la forme d'une tige précontrainte par ressort, au niveau d'un côté frontal de laquelle un ressort (106) applique une force de précontrainte orientée axialement et au niveau de l'autre côté frontal de laquelle une arête d'écrasement (108) est formée.

14. Dispositif d'arrêt de sang selon la revendication 13, **caractérisé en ce qu'**est réalisé dans une section de la tige opposée de manière axiale au ressort (106) un élément de prise (120) sous la forme d'une baguette de maintien, au niveau de laquelle le levier d'enclenchement (110) ou le bouton d'enclenchement s'engage à la manière d'un mécanisme à levier coudé pour faire coulisser la tige dans la première position quand le levier de déclenchement (110) ou le bouton de déclenchement est pivoté contre le côté de boîtier.

15. Dispositif d'arrêt de sang selon l'une quelconque des revendications précédentes, **caractérisé en ce que** sont réalisés au niveau du boîtier (100) de part et d'autre des coussins d'appui pour peau de patient (112), dont les surfaces d'appui tournées vers la peau du patient sont pourvues de bandes autocollantes.
